# FASCICULE DE BREVET EUROPEEN

(11) **EP 2 032 084 B1**
(45) Date de publication et mention de la délivrance du brevet: **15.12.2010**
(21) Numéro de dépôt: 06831114.1
(22) Date de dépôt: 14.11.2006
(51) Int. Cl.: A61F 2/44

(54) **PROTHESE DE DISQUE INTERVERTEBRAL**
BANDSCHEIBENPROTHESE
INTERVERTEBRAL DISC PROSTHESIS

(30) Priorité: 16.11.2005 FR 0511596
(43) Date de publication de la demande: 11.03.2009
(73) Titulaire: Warsaw Orthopedic, Inc., Warsaw, IN 46581 (US)
(72) Inventeur: Pointillart, Vincent, 33000 Bordeaux (FR); Assaker, Richard, 7540 Kain (BE)
(74) Mandataire: Kühn, Armin
(86) Numéro de dépôt international: PCT/FR2006/002515
(87) Numéro de publication internationale: WO 2007/057555

(56) Documents cités:
- US-A1- 2003 187 506
- US-A1- 2005 165 485
- US-B1- 6 419 706
- US-B1- 6 582 466
- US-B1- 6 679 914

## Description

L'invention concerne une prothèse selon le préambule de la revendication1. Ainsi, l'invention concerne une prothèse de disque intervertébral destinée à remplacer partiellement ou totalement les disques naturels assurant la liaison entre deux vertèbres de la colonne vertébrale, quelle que soit la partie concernée du rachis.

### ARRIERE PLAN DE L'INVENTION

On connaît une prothèse mentionnée ci-dessus, par exemple, de US 2005/165 485 A1. Les disques intervertébraux de la colonne vertébrale sont constitués chacun d'un élément central appelé nucleus pulposus, enfermé dans un enroulement de fibres appelées annulus. Le disque assure la liaison entre deux corps vertébraux et le contrôle des mouvements de flexion, d'inclinaison et de rotation de la colonne vertébrale. Ce disque est amené à se dégrader sous l'effet du temps, des efforts ou de certaines maladies dégénératives, il en résulte alors un tassement et/ou un mauvais fonctionnement de celui-ci. Ceci peut entraîner différents types de pathologies, occasionnant de multiples douleurs plus ou moins intenses ainsi que des handicaps plus ou moins importants.

Le traitement de ce type d'affection consiste à pratiquer l'ablation du disque malade et son remplacement soit par un élément mobile ou déformable soit par un élément qui relie rigidement les deux vertèbres concernées.

Plusieurs types de prothèses ont été proposés pour remplacer le disque intervertébral mais elles ne sont que partiellement satisfaisantes. En effet, elles préservent la mobilité intervertébrale et restaure la distance intervertébrale à une valeur proche de celle assurée par un disque sain. En revanche elles imposent, dans cette mobilité, une cinématique particulière qui n'est pas compatible ou qui n'est que partiellement compatible avec la mobilité naturelle relative de deux vertèbres. La prothèse, dans la plupart des cas, impose une cinématique qui lui est propre, avec son centre de rotation et ses différents guidages plan sur plan, qui ne manque pas de venir en interférence avec les éléments de l'articulation naturelle qui demeurent entre deux vertèbres, notamment les facettes articulaires postérieures. Il faut noter à cet égard que le soin avec lequel on doigt poser la prothèse est important car toute imprécision dans ce placement augmente la gravité du conflit entre la cinématique de la prothèse et la cinématique naturelle. Cette mobilité non physiologique peut être à l'origine de conséquences cliniques indésirables. On peut même craindre des risques de migration d'un composant ou de luxation de l'articulation prothétique.

De plus, pour la plupart d'entre elles, les prothèses connues ne sont pas de nature à restaurer la lordose cervicale ou lombaire normale. La restauration de la distance intervertébrale ne prend pas en compte l'inclinaison nécessaire d'une vertèbre à l'autre dans l'empilement qui conduit à cette lordose dont l'existence est utile à une biomécanique normale de l'ensemble du rachis, et plus particulièrement des étages adjacents.

Par ailleurs, les prothèses connues ne sont pas adaptées à absorber les chocs. La conséquence de cette inaptitude, associée au conflit entre la cinématique naturelle et la cinématique de l'articulation, peut conduire à la production d'une usure prématurée, et des éléments naturels, et des éléments prothétiques, risquant de dégrader l'état clinique du patient.

### OBJET DE L'INVENTION

La présente invention entend remédier à ces inconvénients par une prothèse discale de structures simple et de pose facilitée.

### RESUME DE L'INVENTION

À cet effet, la présente invention a pour objet une prothèse de disque intervertébral qui comporte :
- un plateau supérieur rigide,
- un plateau inférieur rigide,
- un coussin, intermédiaire élastiquement compressible, logé entre les surfaces internes des deux plateaux, caractérisée en ce que le coussin est divisé dans le sens de l'épaisseur en deux unités reposant l'une sur l'autre par des surfaces de contact complémentaires, et en ce que le coussin est un composant replié sur lui-même.

Cette structure se présente sous la forme d'une pièce qui n'impose aux deux vertèbres qu'elle relie aucune liaison contrainte (bien entendu pour des amplitudes correspondant à un déplacement relatif naturel). Il s'ensuit que les guides naturels de ce déplacement relatif restent prépondérants (facettes articulaires postérieures notamment) et leur intégrité est préservée. En outre, au niveau de ces surfaces de contact, il est possible par le choix approprié de leur forme et de leur état, de maîtriser la nature et la concentration des contraintes ainsi que les déformations qui existeront à leur niveau.

Les surfaces de contact complémentaires seront, dans un premier mode de réalisation de l'invention, ménagée dans l'épaisseur même du coussin intermédiaire et de préférence sous la forme de creux et de bosses (dents), les bosses de l'une étant complémentaires des creux de l'autre de sorte qu'ils viennent en contact les unes avec les autres par au moins la surface de leurs flancs.

Dans un mode de réalisation préféré de l'invention, le coussin intermédiaire élastiquement compressible, logé entre les deux surfaces internes des plateaux, possède à l'état libre, la forme générale d'un coin entre ces deux plateaux. Cette forme en coin de la structure permet de restaurer la lordose du rachis que la dégénérescence discale a en général détériorée.

Le coussin est par exemple à base de polyéthylènes haute densité de polyuréthanes haute densité, de silicones ou de composites de ces différents matériaux.

La prothèse comporte de manière préférée une membrane de contention du coussin fixée aux plateaux. Cette membrane de contention permet d'éviter l'envahissement biologique et la colonisation de la prothèse. Il assure par ailleurs, bien que tous les matériaux utilisés soient biocompatibles, un isolement complet du coussin à l'égard du milieu biologique voisin et forme une barrière à la migration dans l'organisme d'éventuelles particules provenant du coussin.

Cette membrane peut-être un anneau fixé aux plateaux (laissant un contact direct coussin-plateau), ou bien un sac hermétique, enfermant le coussin, et par exemple collé aux plateaux, ou encore une membrane prise en sandwich entre chaque plateau et une platine rivetée à celui-ci, l'autre face de cette platine étant en contact avec le coussin.

D'autres dispositions constructives apparaîtront à la lecture de ce qui suit. On mentionnera en particulier l'existence d'un lien entre les deux plateaux de la prothèse qui permet de la maintenir dans un état comprimé afin d'en faciliter la pose. L'un des avantages de cette prothèse réside dans la pose aisée de celle-ci qui ne requiert pas une grande précision puisque elle n'impose pas de devoir « accorder » ses degrés de liberté avec ceux, naturels, existant entre deux vertèbres.

### BREVE DESCRIPTION DES DESSINS

D'autres caractéristiques et avantages de l'invention ressortiront de la description donnée ci-après de quelques exemples de sa réalisation.

### DESCRIPTION DETAILLEE DE L'INVENTION

Il sera fait références aux dessins annexés parmi lesquels :
- la figure 1 représente une prothèse selon l'invention placée entre deux vertèbres cervicales,
- la figure 2 est une vue éclatée d'une réalisation d'une prothèse conforme à l'invention,
- la figure 3 est une vue de la prothèse de la figure 2 refermée et maintenue comprimée par un lien sécable,
- la figure 4 est une représentation d'un mode de réalisation du coussin compressible,
- la figure 5 est une représentation d'une variante de réalisation du coussin compressible de la figure précédente,
- la figure 6 est une représentation du coussin de la figure précédente refermé sur lui-même.

### DESCRIPTION DETAILLEE DE L'INVENTION

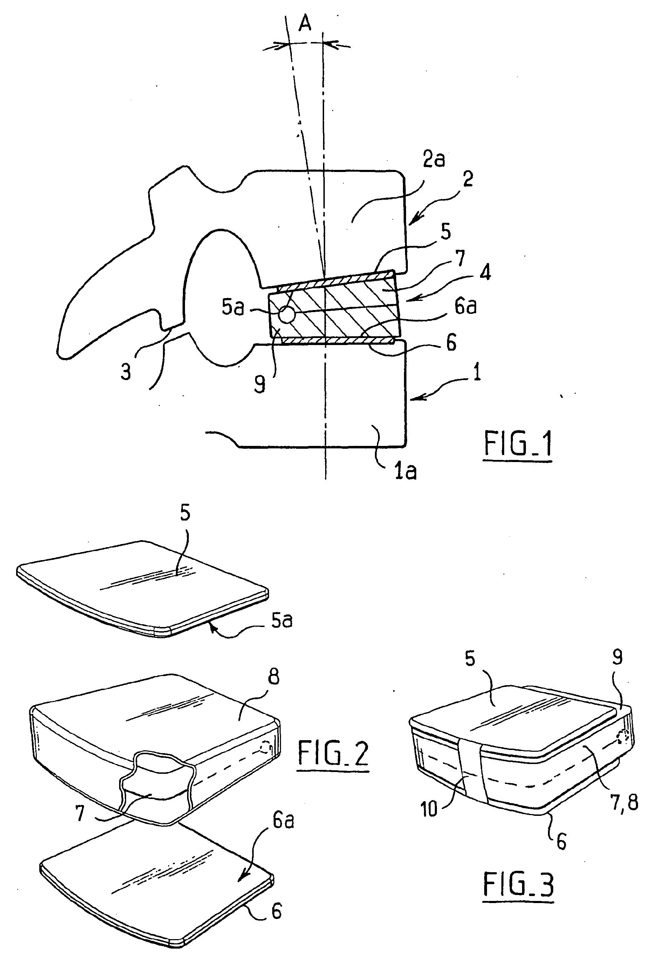

À la figure 1, on a représenté deux vertèbres cervicales successives 1 et 2 avec chacune un corps vertébral la et 2a, et une surface articulaire postérieure 3. Entre les corps vertébraux, on a représenté, de manière schématique, une prothèse 4 qui comporte deux plateaux extrêmes 5 et 6 et un corps intermédiaire 7 en forme de coussin compressible qui a la forme d'un coin dont le sommet ou l'arête (l'extrémité la moins épaisse) est postérieur, la partie de droite de la figure représentant la zone antérieure du rachis.

Les plateaux 5 et 6 extrêmes de la prothèse sont au contact des corps vertébraux la et 2a par l'intermédiaire de surfaces préalablement préparées sur ces corps après ablation du disque naturel détérioré. La surface externe de ces plateaux peut comporter des moyens d'ancrage, par exemple des reliefs, pour améliorer la fixation à l'os ; il peut également s'agir de vis ou tout élément équivalente. On a représenté la lordose du rachis par l'angle A qu'impose la prothèse à l'orientation relative des deux vertèbres.

À la figure 2, on constate que chacun des plateaux 5 et 6 possède une surface interne 5a, 6a, ici représentée plane mais qui peut affecter une forme concave sensiblement sphérique ou cylindrique. Le coussin compressible 7 est fixé aux plateaux. Ce coussin est enfermé dans une membrane de contention 8, qui isole biologiquement ce coussin du reste du corps du patient. La fixation de la membrane au reste de la prothèse sera réalisée par tout moyen approprié. On citera par exemple une membrane « cylindrique » fixée au bord de chaque plateau (par sertissage notamment); la membrane peut également être telle que son bord est pris en sandwich entre la face interne de chaque plateau et une platine rapportée sur cette dernière par exemple par rivetage ; elle peut être en forme de sac entièrement fermé sur lequel sont collés les plateaux.

À la figure 3, on a représenté la prothèse dans son état prêt à être insérée entre deux vertèbres, cet état correspondant à une compression du coussin compressible 7 maintenue grâce à un lien 10 disposé sur la face antérieure de la prothèse 4 et qui peut être rompu après mise en place de cette dernière entre les deux corps vertébraux la et 2a. Ce lien peut aussi être disposé de manière à entourer la totalité de la prothèse de sorte que sa rupture et son retrait après coupe si nécessaire, peuvent être réalisés par le chirurgien par voie postérieure.

On remarque sur cette figure que les plateaux 5 et 6 sont de dimensions telles qu'ils sont, dans la partie postérieure de la prothèse, en retrait du coussin, lequel déborde des plateaux par une partie 9 du côté de l'arête du coin.

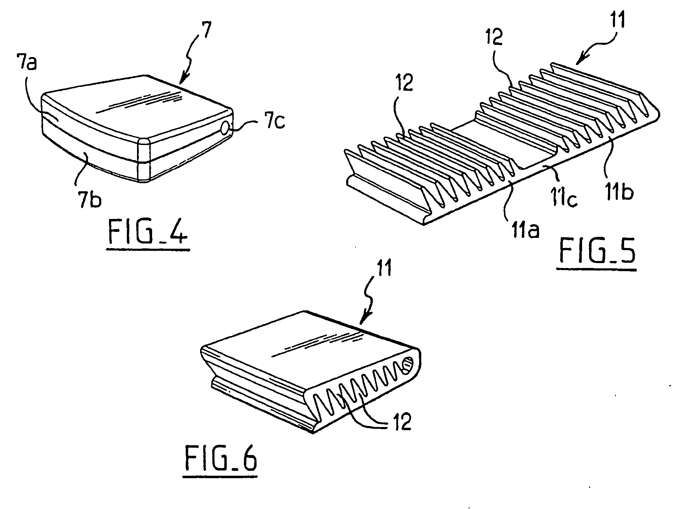

La figure 4, par un détail des figures précédentes, montre un coussin élastiquement compressible 7 repliable en une seule pièce, avec deux parties 7a et 7b repliées l'une sur l'autre autour d'une partie 7c qui constitue une zone amincie formant charnière de pliage qui deviendra l'extrémité postérieure du coin. La prothèse est ainsi divisée en deux unités coopérant l'une avec l'autre par des surfaces de contact complémentaires, ici schématiquement planes.

À la figure 5, le coussin 11 représenté possède également deux parties 11a et 11b repliables l'une sur l'autre autour d'une zone amincie 11c faisant office de charnière de pliage de sorte que, comme représenté à la figure 6, des indentations 12 peuvent s'interpénétrer lorsque les deux parties sont rabattues l'une sur l'autre. On comprend que la surface de contact par laquelle coopérent les deux unités de la prothèse est ici complexe : il s'agit des flancs des dents qui s'interpénètrent. En fabrication, on peut choisir l'angle d'inclinaison de ces indentations 12, leur nombre, leur profil, ... en fonction des caractéristiques élastiques et de comportement souhaitées du coussin.

Dans une variante non représentée, les indentations sont courbes, par exemple,en arc de cercle centré du côté de la charnière.

Dans une autre variante non représentée, les deux parties 11a et 11b peuvent être séparées (la charnière étant supprimée) et simplement superposées avec imbrication de leur indentation.

De manière avantageuse, au moins l'un des plateaux 5 et 6 et de préférence les deux, est dimensionné de façon que la prothèse ait un coussin qui déborde des plateaux à l'arrière de celle-ci pour laisser un espace voisin de la partie postérieure du rachis dépourvu de tout élément métallique avec deux objectifs :
- limiter l'artefact (le brouillage de l'imagerie) de cette partie postérieure du rachis en éloignant de cette partie la partie métallique que constitue, le plateau,
- faciliter la décompression (résection des coins postérieurs des plateaux vertébraux).

On mentionnera enfin le fait que la prothèse ci-dessus décrite correspond à une prothèse monobloc qui est mise en place au moyen d'une voie d'accès antérieure. Dans un mode de réalisation adapté à une mise en place par une voie postérieure, elle comprendra deux demi prothèses qui seront mises en place par des voies d'accès latérales de manière à encadrer latéralement une partie (nucleus pulposus) restante du disque naturel. La forme de chaque demi prothèse sera adaptée à la morphologie rencontrée en étant par exemple, vue de dessus, semblable à un haricot. Chaque demi prothèse sera divisée en deux unités dans les différentes variantes décrites ci-dessus.

## Revendications

1. Prothèse (4) de disque intervertébral comportant :
- un plateau supérieur rigide (5),
- un plateau inférieur rigide (6),
- un coussin intermédiaire (7) élastiquement compressible, logé entre les deux surfaces internes des plateaux (5,6), **caractérisée en ce que** le coussin est divisé dans le sens de l'épaisseur en deux unités reposant l'une sur l'autre par des surfaces de contact complémentaires, et **en ce que** le coussin (7) est un composant replié sur lui-même.

2. Prothèse selon la revendication 1, **caractérisée en ce qu'**elle a à l'état libre, la forme générale d'un coin entre ces deux plateaux.

3. Prothèse selon l'une des revendications précédentes, **caractérisée en ce qu'**elle comporte une membrane (8) de contention du coussin (7) liée aux plateaux (5, 6).

4. Prothèse selon l'une des revendications précédentes, **caractérisée en ce qu'**elle comporte un lien (10) de maintien du coussin à l'état comprimé, susceptible d'être sectionné après la mise en place de la prothèse.

5. Prothèse selon l'une des revendications précédentes **caractérisée en ce que** les surfaces de contact complémentaires des deux unités sont pourvues d'indentations (12) imbriquées.

6. Prothèse selon l'une des revendications précédentes, **caractérisée en ce que** le coussin (7,11) est en deux parties (7a, 7b, 11a 11b) réunies par une zone amincie (7c, 11c) formant charnière de pliage.

7. Prothèse selon l'un des revendications précédentes, **caractérisée en ce que** les plateaux (5, 6) sont limités en partie arrière, la prothèse comportant ainsi une partie (9) postérieure de coussin débordant desdits plateaux.

## Claims

1. Intervertebral disk prosthesis (4), comprising:
- a rigid upper plate (5),
- a rigid lower plate (6),
- an elastically compressible intermediate cushion (7) arranged between the two internal surfaces of the plates (5, 6), **characterized in that** the cushion is divided in the thickness direction into two units resting one upon the other by complementary contact surfaces, and **in that** the cushion (7) is a component folded up onto itself.

2. Prosthesis according to claim 1, **characterized in that** it has, in the released condition, the general shape of a wedge between the two plates.

3. Prosthesis according to any one of the preceding claims, **characterized in that** it comprises a membrane (8) for retaining the cushion (7), connected to the plates (5, 6).

4. Prosthesis according to any one of the preceding claims, **characterized in that** it comprises a link (10) for maintaining the cushion in the compressed condition, capable of being cut after the prosthesis having been arranged in place.

5. Prosthesis according to any one of the preceding claims, **characterized in that** the complementary contact surfaces of the two units are provided with nested indentations (12).

6. Prosthesis according to any one of the preceding claims, **characterized in that** the cushion (7, 11) is made up of two portions (7a, 7b, 11a, 11b) which are connected by a reduced zone (7c, 11c) forming a folding hinge.

7. Prosthesis according to any one of the preceding claims, **characterized in that** the plates (5, 6) are limited in their rear portion, the prosthesis thereby comprising a posterior cushion portion (9) protruding from said plates.

## Patentansprüche

1. Bandscheibenprothese (4), aufweisend:
- eine obere steife Platte (5),
- eine untere steife Platte (6),
- ein elastisch komprimierbares Zwischenkissen (7), das zwischen den beiden Innenflächen der Platten (5,6) angeordnet ist, **dadurch gekennzeichnet, dass** das Kissen in der Dickenrichtung in zwei Einheiten aufgeteilt ist, die durch komplementäre Kontaktflächen aufeinanderliegen, und dass das Kissen (7) ein auf sich selbst aufgefaltetes Bauelement ist.

2. Prothese gemäß Anspruch 1, **dadurch gekennzeichnet, dass** sie im freien Zustand im Wesentlichen die Form eines Keils zwischen den beiden Platten hat.

3. Prothese gemäß einem der vorhergehenden Ansprüche, **dadurch gekennzeichnet, dass** sie eine mit den Platten (5, 6) verbundene Membran (8) zum Halten des Kissens (7) aufweist.

4. Prothese gemäß einem der vorhergehenden Ansprüche, **dadurch gekennzeichnet, dass** sie eine Verbindung (10) zum Halten des Kissens im Kompressionszustand aufweist, das nach dem Einsetzen der Prothese durchtrennt werden kann.

5. Prothese gemäß einem der vorhergehenden Ansprüche, **dadurch gekennzeichnet, dass** die komplementären Kontaktflächen der beiden Einheiten mit ineinandergreifenden Auszahnungen (12) versehen sind.

6. Prothese gemäß einem der vorhergehenden Ansprüche, **dadurch gekennzeichnet, dass** das Kissen (7, 11) aus zwei Teilen (7a, 7b, 11a, 11b) besteht, die durch einen ein Faltgelenk ausbildenden verjüngten Bereich (7c, 11c) aneinander angeschlossen sind.

7. Prothese gemäß einem der vorhergehenden Ansprüche, **dadurch gekennzeichnet, dass** die Platten (5, 6) im hinteren Abschnitt begrenzt sind, wobei die Prothese **dadurch** einen posterioren Kissenabschnitt (9) aufweist, der von den Platten hervorsteht.
